# EUROPEAN PATENT SPECIFICATION

(11) **EP 3 135 251 B1**
(45) Date of publication and mention of the grant of the patent: **28.11.2018**
(21) Application number: 16161905.1
(22) Date of filing: 23.03.2016
(51) Int. Cl.: A61F 2/30, A61F 2/42

(54) **TALAR IMPLANT AND TOTAL ANKLE REPLACEMENT SYSTEM COMPRISING SUCH A TALAR IMPLANT**
TALAR IMPLANTAT UND FUSSGELENKSVOLLPROTHESE MIT SOLCH EINEM TALARIMPLANTAT
IMPLANT ASTRAGALIEN ET SYSTÈME DE REMPLACEMENT TOTAL D'UNE CHEVILLE COMPRENANT UN TEL IMPLANT ASTRAGALIEN

(30) Priority: 25.08.2015 US 201514835184
(43) Date of publication of application: 01.03.2017
(60) Divisional application: 18200312.9
(73) Proprietor: Wright Medical Technology, Inc., Memphis, TN 38117 (US)
(72) Inventor: DHILLON, Braham K., MEMPHIS, TN 38120 (US)
(74) Representative: Lavoix

(56) References cited:
- US-A1- 2002 055 744
- US-A1- 2006 247 788
- US-A1- 2011 218 648
- US-A1- 2012 010 718

## Description

The present invention relates to an implant for use in a total ankle replacement system, in particular a talar implant. The invention also relates to a total ankle replacement system.

### BACKGROUND

An ankle joint may become severely damaged and painful due to arthritis, prior ankle surgery, bone fracture, osteoarthritis, and/or one or more additional conditions. Options for treating the injured ankle have included anti-inflammatory and pain medications, braces, physical therapy, joint arthrodesis, and total ankle replacement.

Total ankle replacement generally comprises two components - tibial implant and a talar implant. The implants comprise articulation surfaces sized and configured to mimic the range of motion of the ankle joint. For example, the talar implant may comprise an implant sized and configured to mimic the talar dome and the tibial implant may comprise an articulation surface sized and configured to mimic articulation of the tibia. An articulating component may be located between the talar implant and the tibial implant.

US 2002/055744, US 2012/010718, US 2011/218648 and US 2006/247788 disclose such talar implants which have a body provided with one or more holes therethrough: these holes are provided to receive bone screws or similar bone fasteners in order to anchor the body to bone, i.e. to the talus, so as to secure fixation of the talar implant.

Soft tissue around the ankle can also be comprised. For example, soft tissue may be partially or completely disconnected from a bone prior to and/or during a total ankle replacement. When performing an ankle replacement, soft tissue must be anchored back to the bone. If bone is not available, the soft tissue cannot be properly anchored and will continue to deteriorate.

### SUMMARY

The present invention is an implant as defined in claim 1.

In various embodiments, an implant is disclosed. The implant includes a body including an articulation surface and an opposed bone contact surface. The implant defines a predetermined thickness between the articulation surface and the bone contact surface. The body defines at least one anchor hole sized and configured to receive an anchor therethrough. The anchor is configured to couple soft tissue to the body. The anchors are coupled to soft tissue and passed through the at least one anchor hole to anchor the soft tissue to the body of the implant.

In various embodiments, a total ankle replacement system is disclosed. The total ankle replacement system includes a tibial implant sized and configured to couple to a resected tibia and a talar implant sized and configured to couple to a resected talus. The talar implant includes a body having an articulation surface and an opposed bone contact surface. The body defines a predetermined thickness between the articulation surface and the bone contact surface. The body defines at least one anchor hole sized and configured to receive an anchor therethrough. The anchor is configured to couple soft tissue to the body.

In various embodiments, an implant is disclosed. The implant includes a body having an articulation surface and an opposed bone contact surface. The body defines a predetermined thickness between the articulation surface and the bone contact surface. The body defines at least one tissue groove formed thereon sized and configured to receive soft tissue. The tissue groove extends at least a predetermined depth from the articulation surface into the body.

### BRIEF DESCRIPTION OF THE FIGURES

The features and advantages of the present invention will be more fully disclosed in, or rendered obvious by the following detailed description of the preferred embodiments, which are to be considered together with the accompanying drawings wherein like numbers refer to like parts and further wherein:
FIG. 1 illustrates an anatomic view of an ankle joint.
FIG. 2 illustrates one embodiment of an ankle joint having a total ankle replacement system therein.
FIG. 3 illustrates one embodiment of an implant having one or more anchor holes formed therethrough.
FIG. 4 illustrates one embodiment of an implant having one or more anchor holes formed through a body of the implant.
FIG. 5 illustrates one embodiment of an implant having one or more anchor holes formed through a stem.
FIG. 6 illustrates one embodiment of an implant having a soft tissue groove formed thereon.

### DETAILED DESCRIPTION

The description of the exemplary embodiments is intended to be read in connection with the accompanying drawings, which are to be considered part of the entire written description. In the description, relative terms such as "lower," "upper," "horizontal," "vertical," "proximal," "distal," "above," "below," "up," "down," "top" and "bottom," as well as derivatives thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

In various embodiments, the present disclosure generally provides an implant for use with a total ankle replacement system. The implant comprises a body having one or more anchor holes formed therethrough. The anchor holes are configured to receive an anchor and soft tissue therein to couple the soft tissue to the implant. In some embodiments, a groove is formed on the talar implant to receive soft tissue therein. The implant provides a secure anchor to allow soft tissue to be positioned at an anatomically correct and/or desirable position with respect to the implant and a bone.

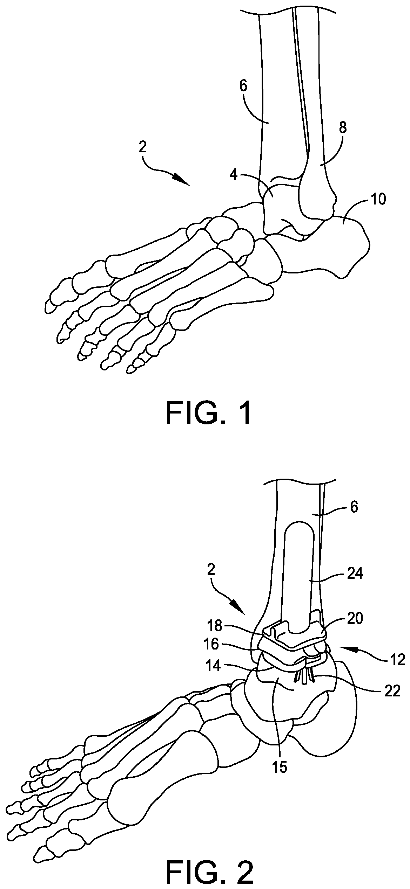

FIG. 1 illustrates an anatomic view of an ankle joint 2. The ankle joint 2 comprises a talus 4 in contact with a tibia 6 and a fibula 8. A calcaneus 10 is located adjacent to the talus 4. In total ankle replacements, the talus 4 and the tibia 6 may be resected, or cut, to allow insertion of a talar implant and a tibial implant. FIG. 2 illustrates the ankle joint 2 of FIG. 1 having a total ankle replacement system 12 inserted therein.

The total ankle replacement system 12 comprises a talar implant 14 and a tibial implant 18. The talar implant 14 comprises a body 15 defining a talar articulation surface 16 (or talar dome). A stem 22 extends into the talus 4 to anchor the talar implant 14 to the talus 4. The tibial implant 18 is sized and configured for installation into the tibia 6. The tibial implant 18 comprises a body 19 having an articulation surface 20 and a tibial stem 24 extending into the tibia 6 to anchor the tibial implant 18. The talar joint surface 16 and the tibial joint surface 20 are mutually sized and configured to articulate. The joint surfaces 16, 20 replace the natural ankle joint surfaces, which are removed, to restore a range of motion that mimics the natural joint. One or more holes may be formed in the tibia and/or the talus prior to and during insertion of the tibial implant 18 or the talar implant 12. For example, in some embodiments, a hole is drilled starting in the bottom of the talus, extending through the talus and into the tibia. The hole may comprise, for example, a 6mm hole configured to receive the stem 24 of the tibial implant 18.

The joint surfaces 16, 20 may be made of various materials, such as, for example, polyethylene, high molecular weight polyethylene (HMWPE), rubber, titanium, titanium alloys, chrome cobalt, surgical steel, and/or any other suitable metal, ceramic, sintered glass, artificial bone, and/or any combination thereof. The joint surfaces 16, 20 may comprise different materials. For example, the tibial joint surface 20 may comprise a plastic or other non-metallic material and the talar joint surface 16 may comprise a metal surface. Those skilled in the art will recognize that any suitable combination of materials may be used.

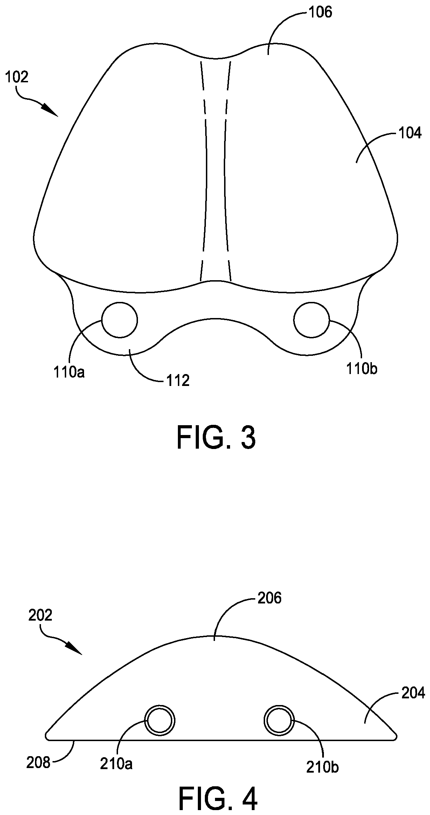

FIG. 3 illustrates one embodiment of a talar implant 102 having a plurality of anchor holes 110a, 110b formed therethrough. The talar implant 102 comprises a body 104 having an articulation surface 106 and an opposed bone contact surface (see FIG. 4). The body 104 has a predetermined thickness between the articulation surface 106 and the bone contact surface. The predetermined thickness can be configured based on the patient (e.g., patient-specific) and/or selected from a range of predetermined thicknesses. The articulation surface 106 is sized and configured to interface with an opposing joint surface of a total ankle replacement system. For example, in one embodiment, the articulation surface 106 is sized and configured to interface with a tibial joint surface, such as, for example, a tibial joint surface 20 as shown in FIG. 2. As another example, the articulation surface 106 may be sized and configured to interface with an articulation implant located between a tibial portion of a total ankle replacement and the implant 106. The articulation surface 106 may comprise any suitable shape, such as, for example, a saddle shape (having two hemispheres) as illustrated in FIG. 3 or a dome shape. The bone contact surface comprises a surface configured to contact a resected bone section. For example, in some embodiments, the bone contact surface is configured to rest on and couple to a resected talus. The bone contact surface may comprise a planar surface, a concave surface, and/or any desirably shaped surface.

The talar implant 102 comprises one or more anchor holes 110a, 110b formed therethrough. The anchor holes 110a, 110b may be formed through any suitable portion of the talar implant 102. For example, in the illustrated embodiment, the anchor holes 110a, 110b are formed through a neck flange 112 coupled to the body 104. In other embodiments, the anchor holes 110a, 110b may be formed through the body 104, for example, through a side of the body 104 and/or through the body 104 from the bone contact surface to the articulation surface 106. The anchor holes 110a, 110b are sized and configured to receive a soft tissue anchor therethrough. The anchor is coupled to soft tissue and couples the soft tissue to the implant 102. For example, in some embodiments, a suture (not shown) is passed through the anchor holes 110a, 110b and through soft tissue to attach the soft tissue to the talar implant 102. In some embodiments, a needle may be coupled to the suture to pass the suture through soft tissue and to couple the soft tissue and the talar implant 102. Any suitable anchor, such as, for example, sutures, staples, tissue tags, and/or any other suitable anchors may be used.

According to the invention, at least one of the anchor holes is also sized and configured to receive soft tissue therethrough. Soft tissue may be threaded through the anchor holes 110a, 110b to anchor the soft tissue to the implant 102. In some embodiments, the soft tissue is retained in the anchor holes 110a, 110b by one or more retention devices, such as, for example, a suture, a tissue tag, a staple, and/or any other suitable retention device. In some embodiments, soft tissue is passed through the anchor holes 110a, 110b and coupled to a bone to maintain the soft tissue in a fixed position.

In some embodiments, the anchor holes 110a, 110b are positioned to align the implant 102, the soft tissue, and a bone. For example, in some embodiments, the anchor holes 110a, 110b are configured to align soft tissue in an anatomically correct and/or desirable position when the soft tissue is coupled to the implant 102. As another example, the soft tissue may be anchored to and used to position the implant 102 prior to coupling the implant 102 to a bone. Although two anchor holes 110a, 110b are illustrated, it will be appreciated that that the implant 102 can comprise any number of anchor holes 110a, 110b, such as, for example, one anchor hole, two anchor holes, three anchor holes, and/or any other suitable number of anchor holes. In embodiments having two or more anchor holes, a first subset of the anchor holes 110a, 110b may be used to anchor soft tissue to the implant 102 and a second subset of the anchor holes 110a, 110b may not be used.

In some embodiments, the anchor holes 110a, 110b are formed through an extension attached to the body 104 of the implant 102. For example, in the illustrated embodiment, the anchor holes 110a, 110b are formed through a neck flange 112 coupled to the body 104. The neck flange 112 is sized and configured to position attached soft tissue in an anatomically correct and/or desirable position with respect to the body 104. For example, in some embodiments, the soft tissue is positioned in a spaced arrangement with respect to the body 104 by the neck flange 112 to mimic the natural placement of the soft tissue and/or to prevent damage to the soft tissue caused by contact with the implant 102, the bone, and/or other structures. Although the illustrated neck flange 12 extends from an anterior portion of the implant 102, it will be appreciated that one or more flanges 112 and/or other extensions may extend from any suitable portion of the body 104 (such as anterior, posterior, lateral, etc.) and may comprise any suitable number of anchor holes 110a, 110b formed therethrough.

FIG. 4 illustrates one embodiment of an implant 202 having a plurality of anchor holes 210a, 210b formed through a body 204 of the implant 202. The implant 202 is similar to the implant 102 discussed with respect to FIG. 1, and similar description is not repeated herein. The implant 202 has a plurality of anchor holes 210a, 210b extending through the body 204 from an articulation surface 206 to a bone contact surface 208. In some embodiments, the anchor holes 210a, 210b are sized and configured to receive one or more anchors therein. The anchors (not shown) are passed through soft tissue located near the implantation site of the implant 202 to couple the soft tissue to the body 204. In some embodiments, the anchor holes 210a, 210b are sized and configured to receive soft tissue therethrough. The soft tissue may be anchored to the body 204 by an suitable retention device, such as, for example, one or more sutures, staples, tissue tags, and/or any other suitable retention device. In some embodiments, one or more anchors and/or soft tissue may be coupled to the bone through the anchor holes 210a, 210b.

Although two anchor holes 210a, 210b are illustrated extending through a lower portion of the body 204, it will be appreciated that any number of anchor holes may be located through any portion of the body 204. For example, in some embodiments, one anchor hole, two anchor holes, three anchor holes, and/or any number of anchor holes are formed through the body 204. The anchor holes 210a, 210b may be located in any suitable arrangement on the body 204. In the illustrated embodiment, the anchor holes 210a, 210b are horizontally aligned along a bottom edge of the body 204. In other embodiments, the anchor holes 210a, 210b may be horizontally aligned, vertically aligned, diagonally aligned, and/or randomly placed on the body 204.

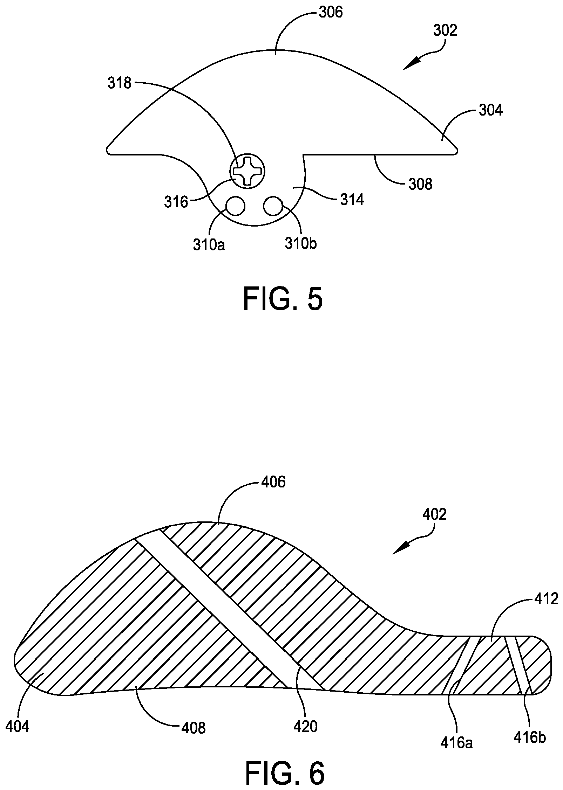

FIG. 5 illustrates one embodiment of an implant 302 having one or more anchor holes 310a, 310b formed through a stem 314. The implant 302 is similar to the implants 104, 204 described with respect to FIGS. 3-4, and similar description is not repeated herein. The implant 302 comprises a stem 314 extending from a bone contact surface 308 of the body 304. The stem 314 is sized and configured to be inserted into a hole formed in a bone, such as, for example, a hole formed in a resected talus during a total ankle replacement. The stem 314 comprises one or more anchor holes 310a, 310b. In some embodiments, the anchor holes 310a, 310b are sized and configured to receive one or more sutures therethrough. In some embodiments, the anchor holes 310a, 310b are sized and configured to receive soft tissue therethrough. The soft tissue is coupled to the body 304 by one or more retention devices, such as, for example, sutures, staples, tissue tags, and/or any other suitable retention device.

In one embodiment, soft tissue is coupled to the implant 302 by passing one or more anchors through the anchor holes 310a, 310b and the soft tissue. A stem 314 of the implant 302 is inserted into a hole formed in a resected talus and anchored to the bone by a bone screw inserted from a first side of the bone, through the fastener hole 316, and into a second side of the bone. The fastener hole 316 and the fastener 318 are configured to couple the implant 302 to a bone, such as, for example, a talus. In some embodiments, the fastener hole 316 may be located through any portion of the implant 302, such as, for example, the stem 314 and/or the body 304. In some embodiments, the fastener hole 316 may comprise internal threading for coupling to external threads of a fastener 318 inserted through the fastener hole 316. In some embodiments, soft tissue is coupled to the stem 314 using the one or more anchor holes 310a, 310b prior to installation of the implant 302 in a bone. When the stem 314 is inserted into a reamed hole in the bone, the soft tissue coupled to the stem 314 is drawn into the hole and coupled to the bone. In some embodiments, one or more access holes are formed through the bone to allow soft tissue and/or anchors to be passed through holes 310a, 310b after the implant 302 is installed in the bone.

FIG. 6 illustrates a cross-section of one embodiment of an implant 402 having a tissue groove 420 formed thereon. The implant 402 is similar to the implants 102, 202, 302 described in conjunction with FIGS. 3-5, and similar description is not repeated herein. The implant 402 comprises a body 404 having a tissue groove 420 formed thereon. The tissue groove 420 is sized and configured to receive soft tissue therein. In some embodiments, the tissue groove 420 is configured to position soft tissue in an anatomically correct and/or desirable position when the implant 402 is coupled to a bone. For example, in some embodiments, the tissue groove 420 is configured to position soft tissue in a position corresponding to a natural placement of the soft tissue in a joint, such as, for example, an ankle joint. In some embodiments, the tissue groove 420 is configured to position the soft tissue such that the soft tissue is not damaged by contact with the implant 402 and/or a bone section.

In some embodiments, the tissue groove 420 is configured to protect soft tissue from damage. The tissue groove 420 extends from the articulation surface 406 a predetermined depth into the body 404 of the implant 402. For example, the tissue groove 420 can comprise a depth such that soft tissue inserted into the tissue groove 420 to be located at or below the surface of the body 404. By placing tissue at or below the surface of the body 404, the tissue groove 420 protects soft tissue from damage due to rubbing, abrasions, and/or other interactions with a bone and/or an implant. Although a single tissue groove 420 is illustrated, it will be appreciated that multiple tissue grooves 420 may be formed on the body 404. In some embodiments, the tissue groove 420 extends from the articulation surface 406 to the bone contact surface 408. In some embodiments, soft tissue and/or anchors can be coupled to the bone through the tissue groove 420.

In some embodiments, the implant 402 includes a neck flange 412 coupled to the body 404. The neck flange 412 extends from an anterior portion of the body 404 and is configured to bear against a neck of a bone, such as, for example, a talus. The neck flange 418 includes one or more fastener holes 416a, 416b formed therethrough. In some embodiments, one or more fasteners are inserted through the fastener holes 416a, 416b to anchor the implant 402 to a bone. For example, in some embodiments, a bone screw is inserted through each of the one or more fastener holes 416a, 416b to anchor the implant 402 to a bone. In some embodiments, one or more anchor holes (not shown) are formed through the body 404 and/or the neck flange 412.

In various embodiments, an implant is disclosed. The implant includes a body including an articulation surface and an opposed bone contact surface. The implant defines a predetermined thickness between the articulation surface and the bone contact surface. The body defines at least one anchor hole sized and configured to receive an anchor therethrough. The anchor is configured to couple soft tissue to the body. The anchors are coupled to soft tissue and passed through the at least one anchor hole to anchor the soft tissue to the body of the implant.

In some embodiments, the body defines one or more fastener holes therethrough sized and configured to receive a fastener. The at least one anchor hole can be sized and configured to receive soft tissue therethrough. The soft tissue is coupled to the anchor. In some embodiments, the body comprises a stem extending from the bone contact surface of the body. The at least one anchor hole is formed through the stem. In some embodiments, the body comprises a neck flange extending from an anterior portion of the body. The at least one anchor hole is formed through the neck flange.

In some embodiments, the body defines one or more tissue grooves formed thereon. The one or more tissue grooves can include a channel formed in the body, wherein the channel has a depth such that soft tissue placed in the channel is at or below an edge of the channel. The one or more tissue grooves can extend from an articulation surface to a bone contact surface.

In various embodiments, a total ankle replacement system is disclosed. The total ankle replacement system includes a tibial implant sized and configured to couple to a resected tibia and a talar implant sized and configured to couple to a resected talus. The talar implant includes a body having an articulation surface and an opposed bone contact surface. The body defines a predetermined thickness between the articulation surface and the bone contact surface. The body defines at least one anchor hole sized and configured to receive an anchor therethrough. The anchor is configured to couple soft tissue to the body.

In some embodiments, the body defines one or more fastener holes therethrough sized and configured to receive a fastener. The at least one anchor hole is sized and configured to receive soft tissue therethrough. The soft tissue can be coupled to the at least one suture. The body can include a stem extending from the bone contact surface. At least one of the anchor holes is formed through the stem. The body can include a neck flange extending from an anterior portion of the body. At least one of the anchor holes is formed through the neck flange.

In some embodiments, the body defines one or more tissue grooves formed thereon. The one or more tissue grooves can include a channel formed in the body having a depth such that soft tissue placed in the channel is at or below an edge of the channel. The one or more tissue grooves can extend from an articulation surface to a bone contact surface.

In various embodiments, an implant is disclosed. The implant includes a body having an articulation surface and an opposed bone contact surface. The body defines a predetermined thickness between the articulation surface and the bone contact surface. The body defines at least one tissue groove formed thereon sized and configured to receive soft tissue. The tissue groove extends at least a predetermined depth from the articulation surface into the body.

The at least one groove can include a depth such that soft tissue placed in the groove is at or below an edge of the at least one groove. The body can further define at least one fastener hole formed therethrough. In some embodiments, the body defines one or more anchor holes formed therethrough. The anchor holes are sized and configured to receive at least one suture therethrough.

Although the subject matter has been described in terms of exemplary embodiments, it is not limited thereto. Rather, the appended claims should be construed broadly, to include other variants and embodiments, which may be made by those skilled in the art. Features of any of the described embodiments may be combined with features of any of the other described embodiments and still be within the scope of the invention, which is defined by the appended claims.

## Claims

1. An implant for use in a total ankle replacement system, the implant comprising a body (104; 204; 304; 404) comprising an articulation surface (106; 206; 306; 406) and an opposed bone contact surface (208; 308; 408) and defining a predetermined thickness therebetween, the body defining at least one anchor hole (110a, 110b; 210a, 210b; 310a, 310b) sized and configured to receive therethrough an anchor that is configured to be coupled to soft tissue and to couple this soft tissue to the body,
**characterized in that** the at least one anchor hole (110a, 110b; 210a, 210b; 310a, 310b) is also sized and configured to receive therethrough soft tissue, and **in that** the implant further comprises at least one of the said anchor, wherein the at least one anchor is adapted to retain to the body (104; 204; 304; 404) the soft tissue threaded through the at least one anchor hole.

2. The implant of claim 1, wherein the body (304; 404) further defines therethrough one or more fastener holes (316; 416a, 416b) sized and configured to receive a fastener (318) to couple the implant (302; 402) to a bone.

3. The implant of claim 1 or claim 2, wherein the body comprises a stem (314) extending from the bone contact surface (308) of the body (302), and wherein the at least one anchor hole (310a; 310b) is formed through the stem.

4. The implant of claim 1 or claim 2, wherein the body (104; 404) comprises a neck flange (112; 412) extending from an anterior portion of the body, and wherein the at least one anchor hole (110a; 110b) is formed through the neck flange.

5. The implant of any one of claims 1 to 4, wherein the body (404) defines one or more tissue grooves (420) formed thereon.

6. The implant of claim 5, wherein the one or more tissue grooves (420) comprise a channel formed in the body (404), and wherein the channel has a depth such that soft tissue placed in the channel is at or below an edge of the channel.

7. The implant of claim 5 or claim 6, wherein the one or more tissue grooves (420) extend from the articulation surface (406) to the bone contact surface (408).

8. The implant of any one of claims 5 to 7, wherein the one or more tissue grooves (420) extend at least a predetermined depth from the articulation surface (406) into the body (404).

9. The implant of any one of claims 1 to 8, wherein the anchor comprises a suture, a staple and/or a tissue tag.

10. The implant of any one of claims 1 to 9, wherein the at least one anchor consists of a suture, a tissue tag and/or a staple.

11. A total ankle replacement system, comprising:
a tibial implant (18) sized and configured to couple to a resected tibia (6); and
a talar implant (14; 102; 202; 302; 402) sized and configured to couple to a resected talus (4), the talar implant being according to any one of claims 1 to 10.

## Patentansprüche

1. Implantat zur Verwendung in einer Fußgelenksvollprothese, wobei das Implantat einen Körper (104; 204; 304; 404) umfasst, umfassend eine Gelenkfläche (106; 206; 306; 406), die eine gegenüber liegende Knochenkontaktfläche (208; 308; 408) umfasst und eine vorbestimmte Dicke dazwischen definiert, wobei der Körper mindestens ein Ankerloch (110a, 110b; 210a, 210b; 310a, 310b) definiert, das abgemessen und konfiguriert ist, um dadurch einen Anker aufzunehmen, der konfiguriert ist, um an Weichgewebe gekoppelt zu sein und dieses Weichgewebe an den Körper zu koppeln,
**dadurch gekennzeichnet, dass** das mindestens eine Ankerloch (110a, 110b; 210a, 210b; 310a, 310b) ebenfalls abgemessen und konfiguriert ist, um dadurch Weichgewebe aufzunehmen, und dadurch, dass das Implantat weiter mindestens eines des Ankers umfasst, wobei der mindestens eine Anker angepasst ist, um am Körper (104; 204; 304; 404) das Weichgewebe zurückzuhalten, das durch das mindestens eine Ankerloch gefädelt ist.

2. Implantat nach Anspruch 1, wobei der Körper (304; 404) weiter dadurch ein oder mehrere Befestigungslöcher (316; 416a, 416b) definiert, die abgemessen und konfiguriert sind, um eine Befestigungsvorrichtung (318) aufzunehmen, um das Implantat (302; 402) an einen Knochen zu koppeln.

3. Implantat nach Anspruch 1 oder Anspruch 2, wobei der Körper einen Stamm (314) umfasst, der sich von der Knochenkontaktoberfläche (308) des Körpers (302) erstreckt, und wobei das mindestens eine Ankerloch (310a; 310b) durch den Stamm gebildet ist.

4. Implantat nach Anspruch 1 oder Anspruch 2, wobei der Körper (104; 404) einen Halsflansch (112; 412) umfasst, der sich von einem vorderen Abschnitt des Körpers erstreckt, und wobei das mindestens eine Ankerloch (110a; 110b) durch den Halsflansch gebildet ist.

5. Implantat nach einem der Ansprüche 1 bis 4, wobei der Körper (104) eine oder mehrere Gewebefurchen (420) definiert, die daran gebildet sind.

6. Implantat nach Anspruch 5, wobei die eine oder die mehreren Gewebefurchen (420) einen Kanal umfassen, der im Körper (404) gebildet ist, und wobei der Kanal eine Tiefe aufweist, so dass das Weichgewebe, das im Kanal platziert ist, an oder unter einer Kante des Kanals ist.

7. Implantat nach Anspruch 5 oder Anspruch 6, wobei sich die eine oder die mehreren Gewebefurchen (420) von der Gelenkfläche (406) zur Knochenkontaktfläche (408) erstrecken.

8. Implantat nach einem der Ansprüche 5 bis 7, wobei die eine oder die mehreren Gewebefurchen (420) sich mindestens eine vorbestimmte Tiefe von der Gelenkfläche (406) in den Körper (404) erstrecken.

9. Implantat nach einem der Ansprüche 1 bis 8, wobei der Anker eine Naht, eine Klammer und/oder eine Gewebemarkierung umfasst.

10. Implantat nach einem der Ansprüche 1 bis 9, wobei der mindestens eine Anker aus einer Naht, einer Gewebemarkierung und/oder einer Klammer besteht.

11. Fußgelenksvollprothese, umfassend:
ein Schienbeinimplantat (18), das abgemessen und konfiguriert ist, um an ein reseziertes Schienbein (6) zu koppeln; und ein Talarimplantat (14; 102; 202; 302; 402), das abgemessen und konfiguriert ist, um an einen resezierten Talus (4) zu koppeln; wobei das Talarimplantat gemäß einem der Ansprüche 1 bis 10 ist.

## Revendications

1. Implant destiné à être utilisé dans un système de remplacement total de cheville, l'implant comprenant un corps (104 ; 204 ; 304 ; 404) comprenant une surface d'articulation (106 ; 206 ; 306 ; 406) et une surface de contact osseuse (208 ; 308 ; 408) opposée et définissant une épaisseur prédéterminée entre elles, le corps définissant au moins un trou d'ancrage (110a, 110b; 210a, 210b; 310a, 310b) dimensionné et configuré pour recevoir, à travers ce dernier, un ancrage qui est configuré pour être couplé au tissu mou et pour coupler ce tissu mou au corps,
**caractérisé en ce que** le au moins un trou d'ancrage (110a, 110b ; 210a, 210b ; 310a, 310b) est également dimensionné et configuré pour recevoir, à travers ce dernier, le tissu mou, et **en ce que** l'implant comprend en outre au moins l'un desdits ancrage, dans lequel le au moins un ancrage est adapté pour retenir sur le corps (104 ; 204 ; 304 ; 404), le tissu mou enfilé à travers le au moins un trou d'ancrage.

2. Implant selon la revendication 1, dans lequel le corps (304 ; 404) définit en outre, à travers ce dernier, un ou plusieurs trous de fixation (316 ; 416a, 416b) dimensionnés et configurés pour recevoir une fixation (318) afin de coupler l'implant (302 ; 402) à un os.

3. Implant selon la revendication 1 ou la revendication 2, dans lequel le corps comprend une tige (314) s'étendant à partir de la surface de contact osseuse (308) du corps (302), et dans lequel le au moins un trou d'ancrage (310a ; 310b) est formé à travers la tige.

4. Implant selon la revendication 1 ou la revendication 2, dans lequel le corps (104 ; 404) comprend une bride de col (112 ; 412) s'étendant à partir d'une surface antérieure du corps, et dans lequel le au moins un trou d'ancrage (110a ; 110b) est formé à travers la bride de col.

5. Implant selon l'une quelconque des revendications 1 à 4, dans lequel le corps (404) définit une ou plusieurs rainures de tissu (420) formées sur ce dernier.

6. Implant selon la revendication 5, dans lequel les une ou plusieurs rainures de tissu (420) comprennent un canal formé dans le corps (404), et dans lequel le canal a une profondeur de sorte que le tissu mou placé dans le canal est au niveau de ou au-dessous d'un bord du canal.

7. Implant selon la revendication 4 ou la revendication 6, dans lequel les une ou plusieurs rainures de tissu (420) s'étendent de la surface d'articulation (406) à la surface de contact osseuse (408).

8. Implant selon l'une quelconque des revendications 5 à 7, dans lequel les une ou plusieurs rainures de tissu (420) s'étendent au moins sur une profondeur prédéterminée à partir de la surface d'articulation (406) dans le corps (404).

9. Implant selon l'une quelconque des revendications 1 à 8, dans lequel l'ancrage comprend une suture, une agrafe et/ou un marqueur de tissu.

10. Implant selon l'une quelconque des revendications 1 à 9, dans lequel le au moins un ancrage se compose d'une suture, d'un marqueur de tissu et/ou d'une agrafe.

11. système de remplacement total de cheville comprenant :
un implant tibial (18) dimensionné et configuré pour se coupler à un tibia réséqué (6) ; et
un implant astragalien (14 ; 102 ; 202 ; 302 ; 402) dimensionné et configuré pour se coupler à un astragale réséqué (4), l'implant astragalien étant selon l'une quelconque des revendications 1 à 10.
